# EUROPEAN PATENT APPLICATION

(11) **EP 2 263 620 A1**
(43) Date of publication of application: **22.12.2010**
(21) Application number: 09162861.0
(22) Date of filing: 16.06.2009
(51) Int. Cl.: A61F 5/56, A47C 27/00

(54) **Snoring reduction apparatus**

(71) Applicant: Koninklijke Philips Electronics N.V., 5621 BA Eindhoven (NL)
(72) Inventor: The designation of the inventor has not yet been filed
(74) Representative: Golla-Franz, Anke Lucia

(57) **Abstract**

The invention relates to a snoring reduction apparatus (1) comprising a position determination unit (3, 4) for determining a position of the person, an actuation pattern determining unit (5) for determining an actuation pattern, according to which the person is to be actuated, depending on the determined snoring and the determined position of the person, and an actuation unit (6, 7) for actuating the person in accordance with the actuation pattern. Since an actuation pattern is determined not only depending on the determined snoring, but also depending on the determined position of the person, the actuation pattern can be adapted to the current position of the snoring person. The consideration of the position of the person while determining the actuation pattern and the corresponding actuation of the person allow more accurately stimulating a person, in order to more effectively reduce snoring of the person.

## Description

### FIELD OF THE INVENTION

The invention relates to a snoring reduction apparatus and a snoring reduction method. The invention relates further to a snoring reduction computer program.

### BACKGROUND OF THE INVENTION

US 2008/0306396 Al discloses an apparatus for controlling snoring by a person. The apparatus comprises a mechanical vibration sensor for sensing snoring and a stimulus device effective, when sensing snoring, to immediately produce a response in the person tending to interrupt the person's snoring.

This apparatus for controlling snoring has the drawback that the stimulus device may not accurately enough be utilized resulting in waking up the snoring person.

### SUMMARY OF THE INVENTION

It is an object of the present invention to provide a snoring reduction apparatus, wherein an actuation of a person can be improved for more accurately stimulating a person, in order to more effectively reduce snoring of the person.

In an aspect of the present invention a snoring reduction apparatus is provided, wherein the snoring reduction apparatus comprises:
- a snoring determination unit for determining snoring of a person,
- a position determination unit for determining a position of the person,
- an actuation pattern determining unit for determining an actuation pattern, according to which the person is to be actuated, depending on the determined snoring and the determined position of the person,
- an actuation unit for actuating the person in accordance with the
actuation pattern.

Since an actuation pattern, according to which the person is to be actuated, is determined not only depending on the determined snoring, but also depending on the determined position of the person, the actuation pattern can be adapted to the current position of the snoring person. The consideration of the position of the person while determining the actuation pattern and the corresponding actuation of the person allow more accurately stimulating a person, in order to more effectively reduce snoring of the person.

The snoring determination unit comprises, for example, a microphone for acoustically detecting snoring.

In an embodiment, the snoring determination unit is adapted to determine the snoring as a degree of snoring depending on at least one of a number of snoring events within a predefined time interval and an intensity of snoring, wherein the actuation pattern determining unit is adapted to determine the actuation pattern depending on the determined degree of snoring and the determined position of the person. A larger number of snoring events within a predefined time interval and/or a larger intensity of snoring preferentially indicate a larger degree of snoring.

The intensity of snoring is preferentially defined by a signal indicative of the snoring of the person, which is measured by a measuring device like a microphone or a vibration sensor. The measurement of such a signal will be explained in more detail further below.

The snoring determination unit can also determine that the person does not snore, i.e. the snoring determination unit can, for example, determine at least two degrees of snoring, a first degree indicating no snoring and a second degree indicating snoring.

In an embodiment, the snoring determination unit comprises a vibration sensor being adapted such that the person can lie on the vibration sensor, wherein the vibration sensor generates vibration signals indicative of vibrations of the person from which the snoring determination unit determines a snoring of the person, in particular, a degree of snoring of the person. For example, a piezoelectric element like a piezoelectric foil can be used, wherein the person preferentially lies on the piezoelectric element while sleeping for detecting vibrations of the person. If the snoring determination unit comprises a vibration sensor for determining a snoring of the person, the snoring reduction apparatus is preferentially adapted such that for the determination of a snoring of the person only vibration signals are used, which are generated while the person is not actuated by the actuation unit, because otherwise the vibration sensor might detect actuation and the snoring determination unit could misinterpret the vibration signal generated by the actuation unit as snoring. In particular, the snoring reduction apparatus is preferentially adapted to interleave the vibration sensing process and the actuation process in time such that the two processes are not performed simultaneously.

The snoring determination unit is preferentially adapted to detect snoring events based on a signal of a microphone and/or a signal of a vibration sensor. The snoring determination unit is preferentially adapted to determine the degree of snoring depending on the number of these snoring events within a predefined time interval and/or depending on the amplitude of the signal.

Preferentially, a higher degree of snoring is determined, if the number of snoring events within a predefined time interval is larger and/or if the amplitude of the signal is larger. It is further preferred that a degree of snoring is determined indicating no snoring, if the number of snoring events within the predefined time interval is below a predefined no snoring threshold, in particular, if snoring events are not detected at all within the predefined time interval, and/or if the amplitude of the signal is below a predefined no snoring threshold. The predefined time interval is, for example, defined by the last 30 seconds.

The actuation pattern is, for example, a temporal pattern and/or a spatial pattern and/or an amplitude pattern.

A temporal actuation pattern indicates, for example, a sequence in which the person is actuated at one or several locations. In addition or alternatively, the temporal actuation pattern can also indicate the frequency with which a person is actuated at one or several locations. In an embodiment, the frequency depends on the determined degree of snoring. If the degree of snoring decreases indicating that snoring is reduced, the frequency is also decreased.

A spatial actuation pattern indicates preferentially locations at which the person is to be actuated and the amplitude actuation pattern preferentially indicates the amplitude of actuation at one or several locations of the person. In an embodiment, the amplitude of the actuation pattern is determined depending on the determined degree of snoring of the person. In particular, if it is determined that the degree of snoring of a person decreases, the amplitude of actuation is also decreased.

In an embodiment, the actuation pattern determining unit is adapted to determine an actuation pattern having an increasing frequency and/or an increasing amplitude, if the degree of snoring has not been reduced within a predefined time interval, for example, if the degree of snoring has not been reduced within the last 30 seconds.

The actuation unit comprises preferentially a vibration actuator for stimulating the person by vibration in accordance with the determined actuation pattern. In another embodiment, instead of or in addition to the vibration actuator another actuation element can be used for stimulating the person, for example, a temperature element can be used for stimulating the person in accordance with the actuation pattern.

The snoring reduction apparatus is preferentially adapted to perform a feedback mechanism, wherein the snoring and the position of the person are determined as a feedback of the actuation of the person in accordance with the actuation pattern by the actuation unit, wherein this feedback, i.e. the determined snoring and position of the person, are used for updating the actuation pattern by the actuation pattern determining unit, in order to adapt the actuation pattern to the current determined snoring and position of the person, and wherein the actuation unit actuates the person in accordance with the updated actuation pattern. Preferentially, the snoring reduction apparatus is adapted such that the locations, at which the person is actuated, and/or the phase shifts between periodic actuations at different locations are modified, in particular, if the degree of snoring has not been reduced within a predefined time interval, for example, if the degree of snoring has not been reduced within the last 30 seconds.

The snoring reduction apparatus is preferentially adapted such that the actuation of the person stops, if a desired degree of snoring has been reached, in particular, if a degree of snoring has been reached indicating that the person is not snoring anymore.

It is further preferred that the snoring reduction apparatus further comprises a sleep level determination unit for determining the sleep level of the person, wherein the actuation pattern determining unit is adapted to determine the actuation pattern depending on the determined sleep level. This allows adapting the actuation pattern to the sleep level. For example, the sleep level can be related to a probability of waking up the person, if the actuation is performed on the person. Preferentially, the actuation pattern comprises a larger amplitude and/or a larger frequency and/or more locations, at which the person is to be actuated, if the determined sleep level indicates a smaller probability of being waken up by the actuation. For example, if the sleep level determination unit determines a rapid eye movement (REM) level or a light sleep level, an amplitude and/or a frequency and/or a number of locations at which the person is to be actuated can be determined, which is smaller in comparison to an amplitude and/or a frequency and/or a number of locations at which the person is to be actuated, if the determined sleep level indicates a deep sleep level. This further improves the accuracy of stimulating a person, in order to more effectively reduce snoring of the person, wherein the probability of an unwanted awakening is further reduced.

The snoring determination unit and/or the position determination unit and/or the actuation pattern determining unit and/or the sleep level determination unit can be distributed over several units, i.e. these units can be integrated in a single unit or can be constituted by any number of units for fulfilling the corresponding functions.

It is further preferred that the actuation pattern determining unit comprises an assigning unit providing an assignment for assigning the determined snoring of the person and the determined position of the person to an actuation pattern, wherein the actuation pattern determining unit is adapted to determine an actuation pattern in accordance with the assignment. This allows easily determining the respective actuation pattern, depending on the determined snoring of the person and the determined position of the person by using the corresponding assignment. The assignment can be implemented as a look-up table which allows determining the actual actuation pattern very fast in real time. The assignment can also be a function which provides an actuation pattern depending on the determined snoring of the person and the determined position of the person.

The assigning unit can also be adapted to provide an assignment for determining an actuation pattern depending on more parameters, i.e. depending on the determined snoring of the person and the determined position of the person and at least one further parameter like the sleep level. The determined assignment is preferentially stored in the actuation pattern determining unit. Generally, a plurality of assignments is stored in the actuation pattern determining unit for determining an actuation pattern for an actual combination of at least the determined snoring of the person and the determined position of the person.

It is further preferred that the snoring reduction apparatus comprises a change determination unit for determining at least one of a snoring change and a position change and a snoring reduction value determination unit for determining a snoring reduction value indicative of a snoring reduction, wherein the snoring reduction apparatus is adapted to perform following steps for several actuation patterns and a same initial snoring and a same initial position of the person for determining for each of the several actuation patterns a snoring reduction value:
- determining the initial snoring of the person by the snoring determination unit and determining the initial position of the person by the position determination unit,
- actuating the person in accordance with an actuation pattern by the actuation unit,
- determining a resulting snoring of the person by the snoring determination unit and determining a resulting position of the person by the position determination unit,
- determining at least one of a snoring change based on the initial snoring and the resulting snoring and a position change based on the initial position and the resulting position by the change determination unit
- determining a snoring reduction value from at least one of the snoring change and the position change by the snoring reduction value determination unit,
wherein the actuation pattern determining unit comprises an assignment generation unit for generating an assignment for assigning the determined initial snoring of the person and the determined initial position of the person to an actuation pattern, wherein the assignment generation unit is adapted to assign the actuation pattern of the several actuation patterns, for which the snoring reduction value indicating the largest snoring reduction has been determined, to the initial snoring and the initial position.

Although in the previous paragraph a certain comparison algorithm for generating an actuation pattern is described, also other learning algorithms can be used to learn which actuation pattern is most effective.

In an embodiment, the snoring change is determined as the difference between the number of snoring events within a predetermined time interval before and after the person is actuated. In this case, the snoring reduction value is preferentially equal to the snoring change, i.e. equal to the difference in the snoring events within a predetermined time interval before and after the person is actuated. In a further embodiment, the snoring change can be determined as the difference of the intensity of snoring before and after the person is actuated. In this case the intensity is, for example, the average intensity, the maximum intensity, the sum of all intensities or the median intensity within the predefined time period.

In a further embodiment, the snoring reduction value can be a combination like a linear combination of the above mentioned difference in the number of snoring events and the above mentioned difference in intensity within the predefined time interval. A decreasing function can be used for determining a snoring reduction value, wherein the snoring reduction value becomes smaller, if the above mentioned difference in the number of snoring events and/or the above mentioned difference in intensity increases. The decreasing function can, for example, depend on the product or the sum of the above mentioned difference number of snoring events and the above mentioned difference intensity.

Preferentially, the assignment generation unit is adapted to assign the actuation pattern of several actuation patterns, which all have a similar snoring reduction value indicating the largest snoring reduction, to the initial snoring and the initial position, which has the smallest probability of waking up the person. For example, if a first snoring reduction value and a second snoring reduction value are similar and both indicate the largest snoring reduction, the actuation pattern of the first and second actuation patterns, which has the smaller amplitude and/or the smaller frequency of actuation, is used for generating the assignment. The similarity is determined with respect to a similarity measure. According to the similarity measure the first snoring reduction value and the second snoring reduction value are preferentially similar, if their absolute difference is smaller than a predefined threshold which can, for example, be chosen by the person or by a physician. This can lead, for example, to an actuation pattern actuating a person at more sensitive locations, i.e. location being more sensitive with respect to the resulting position change of the person, with a smaller amplitude instead of actuating the person at less sensitive locations with a larger amplitude.

It is further preferred that the position determination unit is adapted to
- determine person locations at which the person is present for determining a location distribution,
- determine a main axis of the location distribution,
- determine one of a left lying position, a right lying position and a prone/supine lying position depending on the location distribution with respect to the main axis,
wherein the assigning unit is adapted to provide assignments to determine an actuation pattern for actuating on:
- the left part of the person, if a left lying position is determined,
- the right part of the person, if a right lying position is determined,
- the left part or the right part of the person, if a prone/supine lying position is determined. This allows moving the person to a prone/supine lying position or right lying position, if the person lies on his/her left side, moving the person to his/her prone/supine lying position or left lying position, if the person is lying on his/her right side, and moving the person to a left lying position or a right lying position, if the person is in a prone/supine lying position, for reducing snoring. For determining person locations at which the person is present for determining a location distribution, the position determination unit is preferentially provided with an array of pressure sensors, which preferentially use the piezoelectric effect, wherein a measured pressure at a location indicates that at this location the person is present. The array of pressure sensors is preferentially provided in a bed, in which the person is lying. In other
embodiments the position determination unit can also be adapted to determine person locations, at which the person is present, for determining a location distribution also by other means like optical means which generates an image of the person and which comprises image processing software for determining the location distribution from different colors, intensities and/or shapes shown in the image.

The determination of a body position based on an image of a camera, in particular, based on an image of an infrared camera, is known from, for example, the article "Artificial neural networks based sleep motion recognition using night vision cameras", Chung-Hsien Kuo et al., Biomedical Engineering: Applications, Basis and Communications, Vol. 16, No. 2 (2004) pages 79 to 86, which is herewith incorporated by reference. Preferentially, two artificial neural networks are used for determining the body position from the image of camera.

It is also preferred that the position determination unit is adapted to
- determine person locations at which the person is present for determining a location distribution,
- determine a main axis of the location distribution,
- determine whether the person is in a prone/supine lying position or not depending on the location distribution with respect to the main axis,
wherein the assigning unit is adapted to provide assignments to determine an actuation pattern for actuating on:
- the left part or right part of the person, if a prone/supine lying position is determined.

In other embodiments, the position determination unit can be adapted to determine whether the person is in a prone/supine lying position or not depending on another property of the location distribution, for example, depending on the shape of the location distribution or by other means like an imaging unit for imaging the person, wherein it is determined from an image of the person whether the person is in an prone/supine lying position or not.

The main axis of the location distribution can, for example, be determined by using a least squares fit. Since the snoring reduction apparatus is preferentially adapted to be sensed in a bed, in which the person sleeps, it is known that the main axis has to be oriented substantially in a direction pointing from the foot site to the head site of the bed or vice versa. Therefore, for determining the main axis by fitting, a straight line extending from the foot site to the head site is preferentially used as an initial straight line.

During fitting the straight line to the location distribution, the person locations can be weighted. Preferentially, if the person locations are determined by using pressure sensors, a person location corresponding to a larger detected pressure receives a larger weight in comparison to the weight of a person location at which a smaller pressure is detected.

It is further preferred that the actuation pattern determining unit is adapted to determine an actuation pattern for actuating the person stronger at the lateral locations than at the more central locations. If it is assumed that a person is less sensitive to actuation at lateral locations in comparison to central locations, actuating the person stronger at the lateral locations than at the more central locations further improves the effectiveness of actuating the person such that snoring is reduced, without or with only a little probability of waking up the person.

In a further aspect to the present invention a snoring reduction method is provided, wherein the snoring reduction method comprises following steps:
- determining snoring of a person,
- determining a position of the person,
- determining an actuation pattern, according to which the person is to be actuated, depending on the determined snoring and the determined position of the person,
- actuating the person in accordance with the actuation pattern.

In a further aspect of the present invention a snoring reduction computer program is provided, wherein the snoring reduction computer program comprises program code means for causing a snoring reduction apparatus as defined in claim 1 to carry out the steps of the snoring reduction method as defined in claim 8, when the computer program is run on a computer controlling the snoring reduction apparatus.

It shall be understood that the snoring reduction apparatus of claim 1, the snoring reduction method of claim 8, and the snoring reduction computer program of claim 9 have similar and/or identical preferred embodiments, in particular, as defined in the dependent claims.

It shall be understood that preferred embodiments of the invention can also be any combination of the dependent claims with a respective independent claim.

### BRIEF DESCRIPTION OF THE DRAWINGS

These and other aspects of the invention will be apparent from and elucidated with reference to the embodiments described hereinafter. In the following drawings
Fig. 1 shows schematically and exemplarily an embodiment of a snoring reduction apparatus,
Figs. 2 to 5 show schematically and exemplarily actuation patterns,
Fig. 6 shows schematically and exemplarily a further embodiment of a snoring reduction apparatus and
Fig. 7 shows a flowchart exemplarily illustrating an embodiment of a snoring reduction method.

### DETAILED DESCRIPTION OF EMBODIMENTS

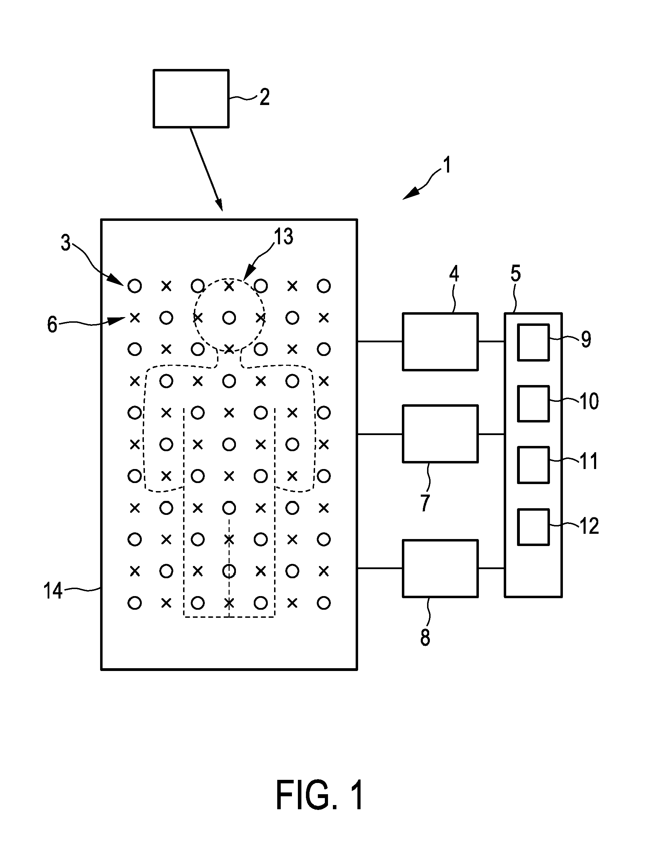

Fig. 1 shows schematically and exemplarily an embodiment of a snoring reduction apparatus. The snoring reduction apparatus 1 comprises a snoring determination unit 2 for determining a snoring of a person 13 lying in a bed 14. The snoring reduction apparatus 1 further comprises an array 3 of pressure sensors, which preferentially use the piezoelectric effect, wherein a measured pressure and a location indicates that at this location the person is present. The pressure sensors are indicated in Fig. 1 by circles and are provided in the bed 14, in which the person 13 is lying. The array 3 of pressure sensors can, for example, be integrated in the mattress or in a sheet of the bed 14.

The snoring reduction apparatus further comprises a position detection unit 4 for detecting the position of the person 13 depending on pressure signals received from the array 3 of pressure sensors. The array 3 of pressure sensors and the position detection unit 4 form a position determination unit for determining a position of the person 13.

In this embodiment, the array 3 of pressure sensors and the position detection unit 4 are adapted to determine locations, at which the pressure sensors of the array 3 of pressure sensors measure a pressure above a predefined threshold, as person locations at which the person is present. The threshold is defined such that it is exceeded if a person lies on the respective pressure sensor. The array 3 of pressure sensors and the position detection unit 4 are further adapted to determine the group of determined person locations as a location distribution.

The position detection unit 4 is further adapted to determine a main axis of the location distribution. In this embodiment, this is performed by using a least squares fit. Since the snoring reduction apparatus 1 is adapted to be used in the bed 14, in which the person 13 sleeps, it is known that the main axis has to be oriented substantially in a direction pointing from a foot site to the head site or vice versa, i.e. the main axis has to be oriented substantially vertically in Fig. 1. Therefore, for determining the main axis by fitting, a straight line extending from the foot site to the head site is used as an initial straight line.

The position detection unit 4 can be adapted to weight the person locations while fitting the straight line to the location distribution. A person location corresponding to a larger detected pressure receives a larger weight in comparison to the weight of a person location, at which a smaller pressure is detected.

In this embodiment, the position detection unit 4 is adapted to determine a left lying position, if the number of person locations on the right side of the main axis is larger than the number of person locations on the left side of the main axis. Correspondingly, the position detection unit 4 is adapted to determine a right lying position, if the number of person locations on the left side of the main axis is larger than the number of person locations on the right side of the main axis, and the position detection unit 4 is preferentially adapted to determine a prone/supine lying position, if the number of person locations on the left side of the main axis is similar to the number of person locations on the right side of the main axis. Preferentially, a person locations number threshold is defined, wherein the position detection unit 4 is adapted to determine a left lying position, if the difference between the number of the person locations on the right side and the number of person locations on the left side of the main axis is larger than the person locations number threshold, and to determine a right lying position, if a difference between the number of person locations on the left side and the number of person locations on the right side of the main axis is larger than the person locations number threshold. If these differences are below the person locations number threshold, a prone/supine lying position is determined. The person locations number threshold can, for example, be determined by calibration, wherein the above mentioned differences are determined while the person is in the left lying position, in the right lying position or in the prone/supine lying position, respectively.

In other embodiments, the position determination unit can also be adapted to determine person locations, in which the person is present, for determining a location distribution also by other means like optical means, which generates an image of the person and which comprises image processing software for determining the location distribution from different colours, intensities and/or shapes shown in the image.

In another embodiment, the array 3 of pressure sensors generates a pressure pattern, wherein at each position within the array 3 of pressure sensors an amount of pressure is measured. This pressure pattern can be used for determining the lying position of the person. For example, this pressure pattern can be used for determining whether the person is lying on his/her left side, right side or whether the person is in a prone/supine lying position. For example, calibration data can be determined by measuring different pressure patterns for different known lying positions of a person. During an actual determination of the lying position, a lying position can be assigned to the current pressure pattern by using the calibration data.

The snoring determination unit 2 comprises a microphone for acoustically detecting snoring. The snoring determination unit 2 is adapted to determine the snoring as a degree of snoring depending on at least one of a number of snoring events within a predefined time interval and an intensity of snoring. In particular, the snoring determination unit 2 is adapted to determine at least two degrees of snoring, a first degree indicating no snoring and a second degree indicating snoring.

The snoring determination unit 2 is adapted to determine a higher degree of snoring, if the number of snoring events within a predefined time interval is larger and/or if the intensity is larger, i.e. the snoring determination unit 2 is preferentially adapted to determine an increasing degree of snoring with an increasing number of snoring events within a predefined time interval and/or with an increasing intensity of snoring. The snoring determination unit 2 is further adapted to determine a degree of snoring indicating no snoring, if the number of snoring events within the predefined time interval is below a predefined no snoring number, in particular, if snoring events are not detected at all within the predefined time interval, and/or if the amplitude of the sensed signal is below a predefined no snoring threshold. The predefined time interval is, for example, defined by the last 30 seconds. The snoring determination unit 2 is adapted to determine a snoring event, if the acoustically sensed signal is larger than a predefined threshold. Also this threshold can be determined by calibration, wherein snoring sounds of a person are acoustically detected.

Instead or of or in addition to the microphone, the snoring determination unit 2 can comprise a vibration sensor being integrated in the bed 14, for example, in a mattress or a sheet of the bed 14, wherein the vibration sensor generates vibration signals indicative of vibrations of the person 13. The snoring determination unit is then preferentially adapted to determine a snoring of the person, in particular, a degree of snoring of the person from the generated vibration signals. For example, a piezoelectric element like a piezoelectric foil can be used as vibration sensor, wherein the person 13 3 preferentially lies on the piezoelectric element while sleeping for detecting vibrations of the person 13. In this embodiment, the snoring determination unit is adapted to detect snoring events based on the vibration signals generated by the vibration sensor, wherein the snoring determination unit is adapted to determine the degree of snoring depending on the number of these snoring events within a predefined time interval and/or depending on the amplitude of the vibration signals generated by the vibration sensor within the predefined time interval.

If the snoring determination unit comprises a vibration sensor for determining a snoring of the person, the snoring reduction apparatus is preferentially adapted such that for the determination of a snoring of the person only vibration signals are used, which are generated while the person is not actuated by the actuation unit, because otherwise the vibration sensor might detect actuation and the snoring determination unit could misinterpret the vibration signal generated by the actuation unit as snoring. In particular, the snoring reduction apparatus is preferentially adapted to interleave the vibration sensing process and the actuation process in time such that the two processes are not performed simultaneously.

The detection of a snoring event from the vibration signal of the vibration sensor is known from, for example, US 2008/0306396 A1, which is herewith incorporated by reference.

The snoring reduction apparatus further comprises an actuation pattern determining unit for determining an actuation pattern, according to which the person 13 is to be actuated, depending on the determined snoring and the determined position of the person 13. In this embodiment, the actuation pattern determining unit 5 is adapted to determine the actuation pattern depending on the determined degree of snoring and the determined position of the person. Moreover, in this embodiment, the actuation pattern determining unit 5 is further adapted to determine the actuation pattern depending on a determined sleep level determined by a sleep level determination unit 8.

The snoring reduction apparatus 1 further comprises an actuation unit for actuating the person in accordance with the actuation pattern.

The actuation pattern is a temporal pattern and/or a spatial pattern and/or an amplitude pattern.

A temporal actuation pattern indicates, for example, a sequence in which the person 13 is actuated at one or several locations. In addition or alternatively, the temporal actuation pattern can also indicate the frequency with which the person 13 is actuated at one or several locations. Preferentially, the frequency depends on the determined degree of snoring. If the degree of snoring decreases indicating that snoring is reduced, the frequency is also decreased.

The spatial actuation pattern indicates locations at which the person 13 is to be actuated and the amplitude actuation pattern indicates the amplitude of actuation and one or several locations of the person 13. Preferentially, the amplitude of the actuation pattern is determined depending on the determined degree of snoring of the person. It is further preferred that the amplitude of actuation is decreased, if it is determined that the degree of snoring of the person is decreased.

The actuation pattern determining unit 4 is adapted to determine an actuation pattern having an increasing frequency and/or an increasing amplitude, if the degree of snoring has not been reduced within a predefined time interval, for example, if the degree of snoring has not been reduced within the last 30 seconds.

The snoring reduction apparatus 1 is adapted to perform a feedback mechanism, wherein the snoring, the position and preferentially the sleep level of the person 13 are determined as a feedback of the actuation of the person 14 in accordance with the actuation pattern by the actuation unit, wherein this feedback, i.e. the determined snoring, position and preferentially sleep level of the person, are used for updating the actuation pattern by the actuation pattern determining unit 5, in order to adapt the actuation pattern to the current determined snoring, position and preferentially sleep level of the person, and wherein the actuation unit actuates the person 13 in accordance with the updated actuation pattern.

The actuation unit comprises vibration actuators indicated in Fig. 1 by crosses. The vibration actuators form an array 6 of vibration actuators for stimulating the person 13 by vibration in accordance with the determined actuation pattern. Also the vibration actuators are integrated in the bed, for example, in the mattress or in a sheet of the bed 14. In another embodiment, instead of the array 6 of vibration actuators or in addition to this array 6 of vibration actuators, other actuation elements can be used for stimulating the person 13, for example, an array of temperature elements can be used for stimulating the person 13 in accordance with the actuation pattern.

The snoring reduction apparatus 1 is adapted such that the actuation of the person 13 stops, if a desired degree of snoring has been reached, in particular, if a degree of snoring has been reached indicating that the person 13 is not snoring anymore. Furthermore, the snoring reduction apparatus 1 is preferentially adapted such that the actuation of the person stops, if the determined sleep level indicates that the person 13 is almost waking up.

The sleep level determined by the sleep level determination unit 8 is related to the probability of waking up the person 13, if the actuation is performed on the person 13. Therefore, the actuation pattern determining unit 5 is preferentially adapted such that the determined actuation pattern comprises a larger amplitude and/or a larger frequency and/or more locations, at which the person 13 is to be actuated, if the determined sleep level indicates a smaller probability of being waken up by the actuation. For example, if the sleep level determination unit 8 determines a REM level or a light sleep level, an amplitude and/or a frequency and/or a number of locations, at which the person 13 is to be actuated, can be determined, which is smaller in comparison to an amplitude and/or a frequency and/or a number of locations, at which the person 13 is to be actuated, if the determined sleep level indicates a deep sleep.

The sleep level determination unit 8 preferentially uses EEG signals for determining the sleep level. This known determination of a sleep level is, for example, disclosed in the article "Roll-over Detection and Sleep Quality Measurement using a Wearable Sensor", Hiroyasu Miwa, Shin-ichiro Sasahara and Toshihiro Matsui, Proceedings of the 29th Annual International Conference of the IEEE EMBS, Cité Internationale, Lyon, France, August 23-26 2007, which is herewith incorporated by reference. In another embodiment, the sleep level determination unit 8 can be adapted to use the time between turns of the person to determine the sleep level, in particular, to distinguish between light and deep sleep. The determination of the sleep level based on the time between turns of the person is also known and, for example, disclosed in the previously cited article. For determining the time between turns of the person, the pressure sensors can be used, wherein a turn could be defined as a position change from a left lying position to a right lying position or vice versa, or from a supine/prone lying position to a right lying position or a left lying position or vice versa.

The actuation pattern determining unit 5 comprises an assigning unit 9 providing an assignment for assigning the determined snoring of the person 13 and the determined position of the person 13 and preferentially also the determined sleep level to an actuation pattern, wherein the actuation pattern determining unit 5 is adapted to determine an actuation pattern in accordance with the assignment. Thus, for different combinations of a determined degree of snoring, a determined position of the person and preferentially of a determined sleep level different assignments to different actuation patterns are provided by the assigning unit 9. These assignments are preferentially implemented as a look-up table, which allows determining the actual actuation pattern very fast in real time. In another embodiment, the assignments can also be stored as a function which provides an actuation pattern depending on the determined snoring, the determined position and preferentially also the determined sleep level.

The snoring reduction apparatus 1 further comprises a change determination unit 11 for determining at least one of a snoring change, a position change and preferentially a sleep level change and a snoring reduction value determination 12 for determining a snoring reduction value indicative of a snoring reduction. The snoring reduction apparatus 1 is adapted to perform following steps for several actuation patterns and a same initial snoring, a same initial position and preferentially a same initial sleep level for determining for each of the several actuation patterns a snoring reduction value:
- determining the initial snoring of the person 13 by the snoring determination unit 2, determining the initial position of the person 13 by the position determination unit 3, 4 and preferentially determining the initial sleep level of the person 13 by the sleep level determination unit 8,
- actuating the person 13 in accordance with an actuation pattern by the actuation unit 6, 7,
- determining a resulting snoring of the person 13 by the snoring determination unit 2, determining a resulting position of the person by the position determination unit 3, 4 and preferentially determining a resulting sleep level of the person 13 by the sleep level determination unit 8,
- determining at least one of a snoring change based on the initial snoring and the resulting snoring, a position change based on the initial position and the resulting position and preferentially a sleep level change based on the initial sleep level and the resulting sleep level by the change determination unit 11,
- determining a snoring reduction value from at least one of the snoring change, the position change and preferentially the sleep level change by the snoring reduction value determination unit 12.

The actuation pattern determining unit 5 further comprises an assignment generation unit 10 for generating an assignment for assigning the determined initial snoring of the person 13, the determined initial position of the person 13 and preferentially the determined initial sleep level of the person 13 to an actuation pattern. The assignment generation unit 10 is adapted to assign the actuation pattern of the several actuation patterns, for which the snoring reduction value indicating the largest snoring reduction has been determined, to the initial snoring, the initial position, and preferentially the initial sleep level. In this way the snoring reduction apparatus 1 provides a learning procedure, wherein different actuation patterns can be tried for reducing snoring, wherein the actuation pattern reducing snoring most effectively can be used for generating a corresponding assignment by the assignment generation unit 10.

The assignment generation unit 10 is adapted to assign the actuation pattern of several actuation patterns, which all have a similar snoring reduction value indicating the largest snoring reduction, to the initial snoring, the initial position and preferentially the initial sleep level, which has the smallest probability of waking up the person. For example, if a first snoring reduction value and a second snoring reduction value are similar and both indicate the largest snoring reduction, the actuation pattern of the first and second actuation patterns, which has the smaller amplitude and/or the smaller frequency of actuation, is used for generating the assignment. The similarity is determined with respect to a similarity measure. According to the similarity measure, the first snoring reduction value and the second snoring reduction value are preferentially similar, if their absolute difference is smaller than a predefined threshold which can, for example, be chosen by the person 13 or by a physician. This can lead to an actuation pattern actuating a person at more sensitive locations, i.e. locations being more sensitive with respect to a resulting position change of the person 13, with a smaller amplitude and/or smaller frequency instead of actuating the person 13 at less sensitive locations with a larger amplitude and/or larger frequency.

In a further embodiment, the assignment generation unit 10 is adapted to assign the actuation pattern of several actuation patterns, which all have a similar snoring reduction value indicating the largest snoring reduction, to the initial snoring, the initial position and preferentially the initial sleep level, which has the least energy consumption and/or the least vibration activation et cetera.

The assigning unit 9 is adapted to provide assignments to determine an actuation pattern for actuating on the left part of the person 13, if a left lying position is determined, on the right part of the person 13, if a right lying position is determined, and on the left part or the right part of the person, if a prone/supine lying position is determined. This allows moving the person 13 to a prone/supine lying position or right lying position, if the person lies on his/her left side, moving the person 13 to his/her prone/supine lying position or left lying position, if the person is lying on his/her right side, and moving the person to a left lying position or a right lying position, if the person is in a prone/supine lying position, for reducing snoring.

The actuation pattern determining unit 5 is adapted to determine an actuation pattern for actuating the person 13 stronger at the lateral locations than at the more central locations.

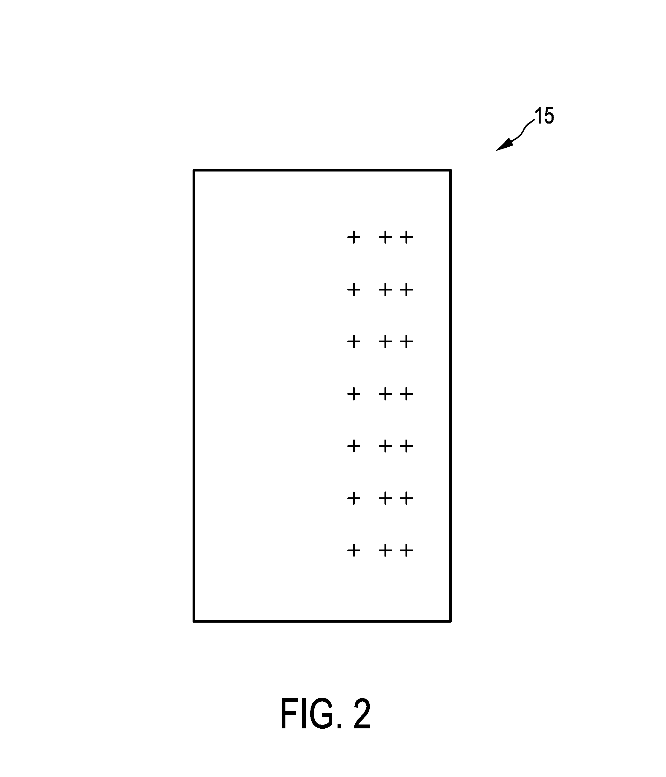

Fig. 2 shows schematically and exemplarily an actuation pattern 15 for actuating a person at the right side of the bed. Double-crosses indicate a larger amplitude in comparison to amplitudes indicated by a single cross. Thus, the actuation pattern 15 shown in Fig. 2 actuates a person stronger at the lateral locations than at the more central locations.

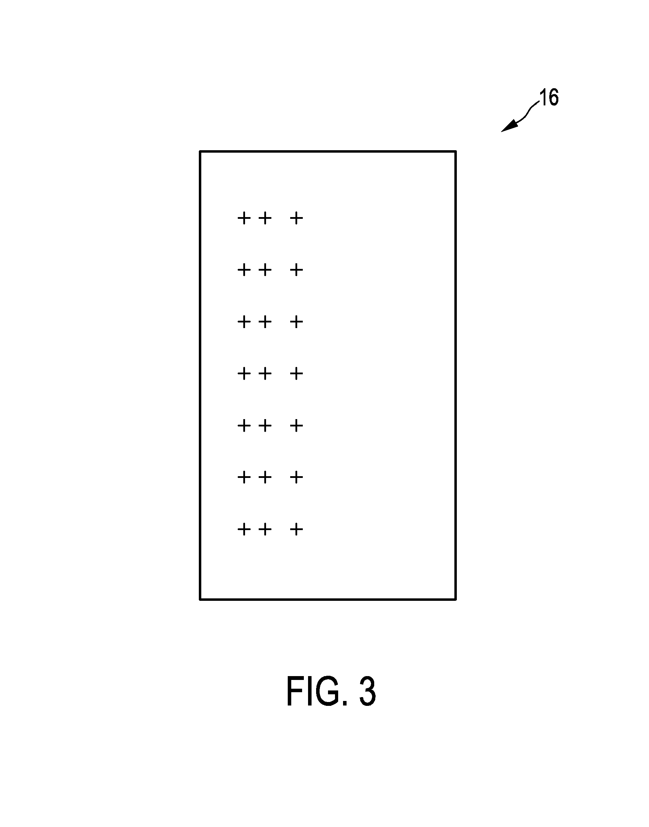

Fig. 3 shows schematically and exemplarily a corresponding actuation pattern 16 for actuating a person at the left side of the bed.

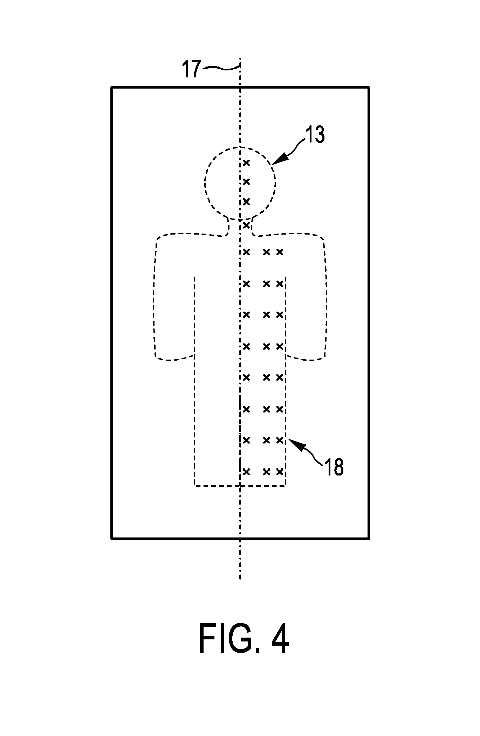

Fig. 4 shows an actuation pattern 18 which considers the position of the person 13. The position determination unit 3, 4 has determined that the person is lying in a prone/supine lying position and the actuation pattern determining unit 5 has determined an actuation pattern 18 which preferentially only activates actuators on the left side of the main axis 17 on which the person 13 is lying, in order to stimulate the person 13 to be transferred into the left lying position, without unnecessarily activating actuators.

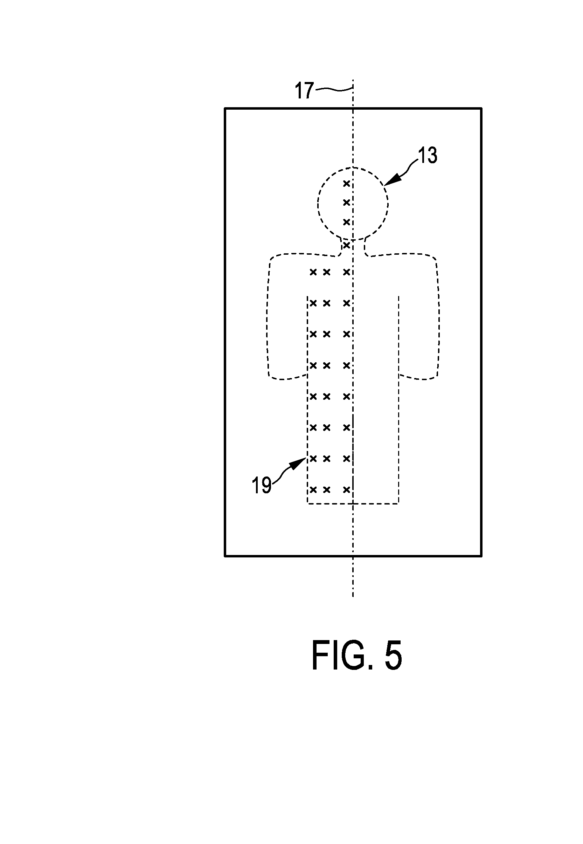

Fig. 5 shows schematically and exemplarily an actuation pattern 19, which considers the position of the person 13, for actuating on the right side of the person 13 for moving the person into a left lying position.

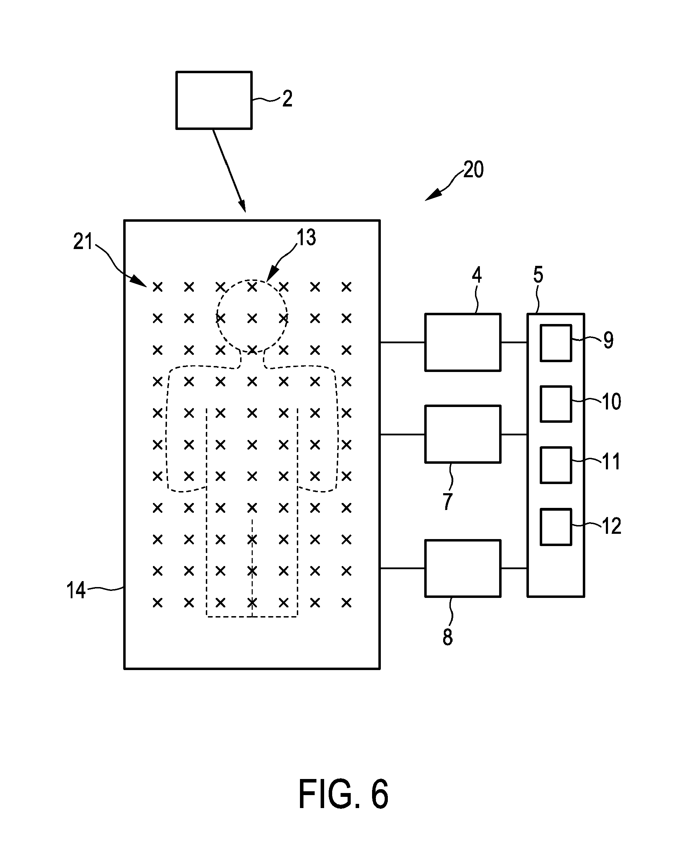

Fig. 6 shows schematically and exemplarily a further embodiment of a snoring reduction apparatus. The snoring reduction apparatus 20 shown in Fig. 6 is similar to the snoring reduction apparatus 1 shown in Fig. 1. The main difference of the snoring reduction apparatus 20 shown in Fig. 6 to the snoring reduction apparatus 1 shown in Fig. 1 is that the snoring reduction apparatus 20 comprises pressure and vibration elements 21, which can detect a pressure indicating that the person 13 is lying on the respective pressure and vibration element and which can actuate on the person 13 by vibration. The pressure and vibration element 21 preferentially comprises a piezoelectric element for detecting pressure and for generating vibration for acting on the person 13.

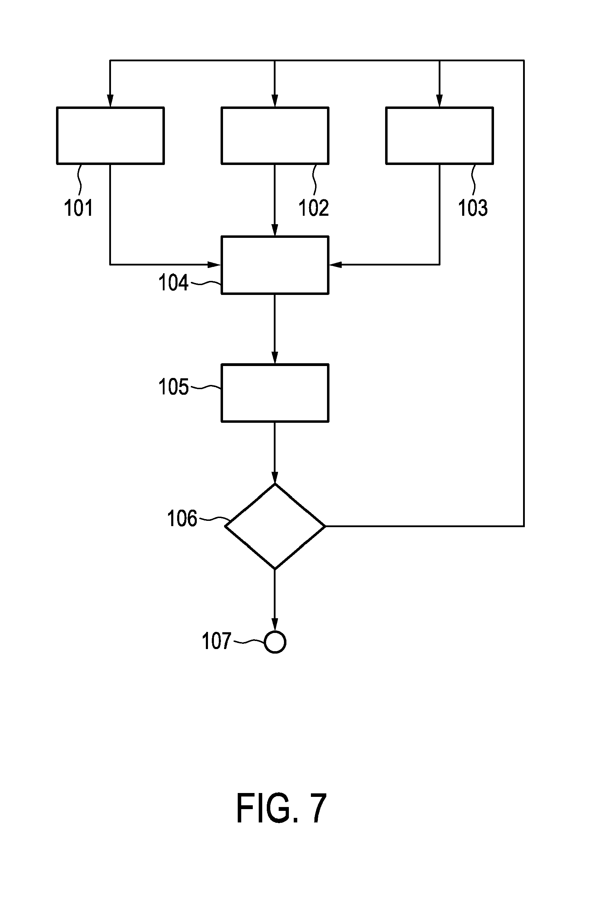

Fig. 7 shows a flowchart exemplarily illustrating a snoring reduction method.

In step 101, a snoring of the person 13 is determined by the snoring determination unit 2. In particular, a degree of snoring of the person 13 is determined by the snoring determination unit 2.

In step 102, the position of the person 13 is determined by the position determination unit 3, 4.

In step 103, the sleep level of the person 13 is determined by the sleep level determination unit 8.

In step 104, an actuation pattern, according to which the person 13 is to be actuated, is determined depending on the determined snoring, the determined position and the determined sleep level by the actuation pattern determining unit 5.

In step 105, the person is actuated in accordance with the actuation pattern by the actuation unit 6, 7.

In step 106, it is determined whether a desired degree of snoring reduction has been reached. If this is the case, the snoring reduction method stops in step 107. Otherwise, the snoring reduction method continues with steps 101 to 103.

Steps 101 to 103 can be performed in any arbitrary sequence. In particular, they can be performed simultaneously.

In another embodiment, step 103 can be omitted.

The snoring reduction apparatus uses preferentially vibrations to trigger the snorer to turn over to his/her side. These vibrations are unobtrusive, while still triggering the snorer to change his/her sleeping position to the side, so that the snoring reduces or even stops completely. As a result, the snoring reduction apparatus also provides a better sleep for people irritated by the snoring sound. The snoring reduction apparatus uses local vibrations to trigger the snorer to change his/her sleeping position. When the snoring reduction apparatus detects, the person is snoring, in particular, severely snoring, the snoring reduction apparatus start vibrations preferentially on only one side of the body. To determine the body position of the snorer, the snoring reduction apparatus uses preferentially contact sensors, in particular, pressure sensors, to measure where the body makes contact with the bed's surface. The position determination unit is preferentially adapted to determine the body position based on the resulting sites, shape and location of the contact area. To escape from the vibrations, the snorer will change his position away from the vibrating motors. The easiest way for the snorer to realize this, is to turn to his/her side. Once the snorer has turned to the side, the snoring will reduce or even stop. Should the person remain on his back, the snoring reduction apparatus will continue to generate vibrations on the determined snorers' body position. The vibration actuators and also further elements can be embedded in bed cover, pillow, belt or clothes.

The vibration actuators, or more generally the mechanical stimulators, are preferentially vibration motors. These motors can be embedded in the mattress cover, bed sheets, or the mattress itself. Additionally, sensors and motors can be embedded in the pillow(s). In a further embodiment, the motors as well as the sensors can be embedded in a belt or other variable device(s) that are worn by the person at one or different locations on his/her body. The motors as well as the sensors can also be integrated into clothes. This would allow the person to use the snoring reduction apparatus even when he/she is not sleeping in his/her own bed.

Each vibration actuator is preferentially activated independently of the others. Vibration frequency and intensity, i.e. amplitude, of each vibration actuator can be controlled. By varying frequency, intensity, and preferentially of the phase of the different vibration actuators, a desired combined actuation pattern, which can also be regarded as a vibration pattern, can be obtained. Depending on the lying position of the person, all or a subset of the vibration actuators are activated. The perceived vibration location can thus be changed by activating and deactivating different vibration motors. In order to prevent adaptation to the vibrations, the vibration intensity, frequency, and location of the active vibration actuators can be varied. Additionally, the intensity can be increased slowly for as long as the snoring continues to increase the motivation for turning to the side. Furthermore, the intensity of the vibration can be adapted to the sleeping phase, i.e. sleep level, for example, REM, or deep sleep, to ensure the snorer senses the vibrations but does not wake up, in particular, does not wake completely.

To prevent unnecessary disturbances, the snoring reduction apparatus preferentially only generates vibrations when the person was snoring in the last x seconds. To detect, whether this is the case, the snoring reduction apparatus comprises the snoring determination unit. If the snoring determination apparatus determines that the person has snored during the last x seconds, the snoring reduction apparatus will generate vibrations on specified locations defined by an actuation pattern that depend on the current detected body position of the snorer. When the snoring reduction apparatus determines that the person has not snored for at least x seconds, no vibrations will be generated. In order to prevent the snoring reduction apparatus from generating only vibrations at intervals corresponding with the snoring, a positive value for parameter x is required. Preferentially, the snoring reduction apparatus generates vibrations as long as a predefined number of snoring events is present in the last x seconds.

For detecting a snoring event, the snoring determination unit can be adapted for using a two-layer neural network as, for example, disclosed in Jane, R.Sola-Soler, J. Fiz, J.A. Morera, J., "Automatic detection of snoring signals: validation with simple snorers and OSAS patients", Engineering in Medicine and Biology Society, 2000, Volume: 4, pp. 3129-3131. Proceedings of the 22nd Annual International Conference of the IEEE, or for using hidden Markov Models as, for example, disclosed W. D. Duckitt, S. K. Tuomi and T. R. Niesler, "Automatic detection, segmentation and assessment of snoring from ambient acoustic data", Physiological Measurement, 2006, Volume: 27, pp. 1047-1056.

Instead of using a pressure sensor for measuring person locations, a vibration actuator for actuating the person and, as described above in an embodiment, a vibration sensor for sensing vibration for determining snoring, a single kind of element, in particular, a piezoelectric element, can be used for fulfilling these functions, i.e. for measuring pressure at a location, for actuating on the person and for sensing vibrations of the person.

Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality.

A single unit or devices may fulfill the functions of several items recited in the claims. The mere fact that certain measures are recited in mutually different dependent claims does not indicate that a combination of these measures cannot be used to advantage.

Determinations like the determination of snoring of a person or of an actuation pattern performed by one or several units or devices, can be performed by any other number of units or devices. The determinations and/or the control of the snoring reduction apparatus in accordance with the snoring reduction method can be implemented as program code means of a computer program and/or as dedicated hardware.

A computer program may be stored/distributed on a suitable medium, such as an optical storage medium or a solid-state medium, supplied together with or as part of other hardware, but may also be distributed in other forms, such as via the Internet or other wired or wireless telecommunication systems.

Any reference signs in the claims should not be construed as limiting the scope.

## Claims

1. A snoring reduction apparatus comprising:
- a snoring determination unit (2) for determining snoring of a person,
- a position determination unit (3, 4) for determining a position of the person,
- an actuation pattern determining unit (5) for determining an actuation pattern, according to which the person is to be actuated, depending on the determined snoring and the determined position of the person,
- an actuation unit (6, 7) for actuating the person in accordance with the actuation pattern.

2. The snoring reduction apparatus as defined in claim 1, wherein the snoring determination unit (2) is adapted to determine the snoring as a degree of snoring depending on at least one of a number of snoring events within a predefined time interval and an intensity of snoring, wherein the actuation pattern determining unit (5) is adapted to determine the actuation pattern depending on the determined degree of snoring and the determined position of the person.

3. The snoring reduction apparatus as defined in claim 1, wherein the snoring reduction apparatus further comprises a sleep level determination unit (8) for determining a sleep level of the person and wherein the actuation pattern determining unit (5) is adapted to determine the actuation pattern depending on the determined sleep level.

4. The snoring reduction apparatus as defined in claim 1, wherein the actuation pattern determining unit (5) comprises an assigning unit (9) providing an assignment for assigning the determined snoring of the person and the determined position of the person to an actuation pattern, wherein the actuation pattern determining unit (5) is adapted to determine an actuation pattern in accordance with the assignment.

5. The snoring reduction apparatus as defined in claim 4, wherein the snoring reduction apparatus comprises a change determination unit (11) for determining at least one of a snoring change and a position change and a snoring reduction value determination unit (12) for determining a snoring reduction value indicative of a snoring reduction, wherein the snoring reduction apparatus is adapted to perform following steps for several actuation patterns and a same initial snoring and a same initial position of the person for determining for each of the several actuation patterns a snoring reduction value:
- determining the initial snoring of the person by the snoring determination unit (2) and determining the initial position of the person by the position determination unit (3,4),
- actuating the person in accordance with an actuation pattern by the actuation unit (6,7),
- determining a resulting snoring of the person by the snoring determination unit (2) and determining a resulting position of the person by the position determination unit (3,4),
- determining at least one of a snoring change based on the initial snoring and the resulting snoring and a position change based on the initial position and the resulting position by the change determination unit (11)
- determining a snoring reduction value from at least one of the snoring change and the position change by the snoring reduction value determination unit (12),
wherein the actuation pattern determining unit (5) comprises an assignment generation unit (10) for generating an assignment for assigning the determined initial snoring of the person and the determined initial position of the person to an actuation pattern, wherein the assignment generation unit (10) is adapted to assign the actuation pattern of the several actuation patterns, for which the snoring reduction value indicating the largest snoring reduction has been determined, to the initial snoring and the initial position.

6. The snoring reduction apparatus as defined in claim 4, wherein the position determination unit (3, 4) is adapted to
- determine person locations at which the person is present for determining a location distribution,
- determine a main axis of the location distribution,
- determine one of a left lying position, a right lying position and a prone/supine lying position depending on the location distribution with respect to the main axis, wherein the assigning unit (9) is adapted to provide assignments to determine an actuation pattern for actuating on:
- the left part of the person, if a left lying position is determined,
- the right part of the person, if a right lying position is determined,
- the left part or the right part of the person, if a prone/supine lying position is determined.

7. The snoring reduction apparatus as defined in claim 1, wherein the actuation pattern determining unit (2) is adapted to determine an actuation pattern for actuating the person stronger at the lateral locations than at the more central locations.

8. A snoring reduction method comprising following steps:
- determining snoring of a person,
- determining a position of the person,
- determining an actuation pattern, according to which the person is to be actuated, depending on the determined snoring and the determined position of the person,
- actuating the person in accordance with the actuation pattern.

9. A snoring reduction computer program comprising program code means for causing a snoring reduction apparatus as defined in claim 1 to carry out the steps of the snoring reduction method as defined in claim 8, when the computer program is run on a computer controlling the snoring reduction apparatus.
